# EUROPEAN PATENT APPLICATION

(11) **EP 2 667 193 A1**
(43) Date of publication of application: **27.11.2013**
(21) Application number: 12305565.9
(22) Date of filing: 23.05.2012
(51) Int. Cl.: G01N 33/574

(54) **MMP2 as a predictive biomarker of response to antiangiogenic therapy and survival after therapy in cancer patients**

(71) Applicant: Université d'Aix-Marseille, 13007 Marseille (FR)
(72) Inventor: The designation of the inventor has not yet been filed
(74) Representative: Leblois-Préhaud, Hélène Marthe Georgette

(57) **Abstract**

The present invention relates to the use of matrix metalloproteinase-2 (MMP2) as a predictive biomarker of response to antiangiogenic therapy and survival after antiangiogenic therapy in cancer patients, and to a related methods for predicting or monitoring the response to an antiangiogenic treatment and the survival after said treatment of a cancer patient.

## Description

The present invention relates to the use of matrix metalloproteinase-2 (MMP2) as a predictive biomarker of response to antiangiogenic therapy and survival after antiangiogenic therapy in cancer patients, and to related methods for predicting or monitoring the response to an antiangiogenic treatment and the survival after said treatment of a cancer patient.

Angiogenesis is a determinant and universal feature associated to tumor growth of solid tumors, and a promising target for cancer treatment. Among many proangiogenic and antiangiogenic factors that regulate angiogenesis, including growth factors, integrins, junction molecules, chemokines and proteases (matrix metalloproteinases or MMPs), vascular endothelial growth factor A (VEGF) has been identified as a major actor of this process (Leung et al., Science, 1989, 246, 1306-1309). Number of antiangiogenic agents that target the VEGF pathway have been successfully developed in the past years and have been approved in the vast majority of cancers (Review in P. Carmeliet and R.K. Jain, Nature, 2011, 473, 298-307; Perren et al., New England Journal of Medicine, 2011, 365, 2484-2496).

Bevacizumab (Avastin), a VEGF-neutralizing monoclonal antibody, was the first antiangiogenic agent that has demonstrated a benefit on progression-free survival (PFS) with or without impact on survival, in patients with advanced and metastatic cancer. This antiangiogenic agent has been approved in the vast majority of cancer, including metastatic colorectal cancer, metastatic non-squamous non-small-cell lung cancer (NSCLC), metastatic renal cell carcinoma (RCC), metastatic breast cancer, ovarian cancer and recurrent glioblastoma (Van Meter, M.E. and E.S. Kim, Curr.Opin. Oncol., 2010, 22, 586-591). With the exception of patients with glioblastoma (GBM), the use of bevacizumab is approved only when combined with cytotoxic or cytokine therapy.

In addition, several multi-targeted tyrosine kinase inhibitors (TKIs) that possess activity against VEGF receptors (VEGFRs) have been approved, including sorafenib (Nexavar) for metastatic RCC and unresectable hepatocellular carcinoma, sunitinib (Sutent) and pazopanib (Votrient) for metastatic RCC, vandetanib (Zactima) for unresectable medullary thyroid cancer, and sunitinib has been recommended for approval for advanced pancreatic neuroendocrine tumors.

As a consequence, an increasing use of antiangiogenic agents has been observed, but limited efficacy and resistance remain outstanding problems, leading to cost issues and reassessment of their benefit. Activity on tumor response, and survival benefit of these agents, vary greatly among patients, as well as with tumor types and agents tested, and biomarkers of efficacy that could identify responders and drive therapeutic decision are missing in oncology (Duda et al., Journal of Clinical Oncology, 2010, 28, 183-185).

Ideal biomarker should be easy to measure on multiple points upon treatment, and standardized in their analysis. Numerous intratumoral or circulating candidate biomarkers have been explored based on their baseline level, their initial variation and/or their changes at progression observed under treatment. However, to date their predictive significance has been generally weak and rarely confirmed among studies. Moreover, some of these candidate biomarkers have been exclusively analyzed in patients treated with antiangiogenic agent, and not compared in patient populations treated without this treatment

Hypertension and polymorphism that affect components of the VEGF pathway have been associated to some predictive value of bevacizumab benefit but not validated to date due to their lack of standardization and an inconsistent effect among tumors (A.M. Jubb and A.L. Harris, Lancet Oncol., 2010, 11, 1172-1183).

In-situ potential biomarkers such as VEGF, VEGF receptor 2 (VEGFR-2) or carbonic anhydrase 9 (CA9) expressions in tumor tissue analyzed on the sample of initial diagnosis have inconsistently been associated with outcome under bevacizumab. High VEGF expression has been correlated to radiographic response, but not to survival, while CA9 seems to modestly impact survival without effect on tumor response (Sathornsumetee et al., J. Clin. Oncol., 2008, 26, 271-278). Another study reported that a high ratio of tumor VEGF-A/VEGFR-2 expression, analyzed at initial diagnosis tend to be associated to a shorter survival (Raizer et al., Cancer, 2010, 116, 5297-5305). Limitation of immunohistochemistry in aheterogeneous tumor tissue, as well as potential discrepancy of biology between initial and recurrent tumor, may explain in part inconsistency of these results. In regard to restricted access to tumor tissue, particularly in brain tumors, a circulating marker is highly desirable to monitor therapy in patients with a brain tumor.

Baseline plasma biomarkers such as VEGF, soluble VEGF receptor 1 (VEGFR-1), placental growth factor (PlGF), stromal cell-derived factor-1 alpha (SDF1-α), vascular cell adhesion protein 1 (VCAM-1), intracellular adhesion molecule 1 (ICAM-1), interleukin 6 (IL-6), interleukin 8 (IL-8), as well as circulating endothelial cells, have been reported to be correlated to outcome under bevacizumab. However, their predictive value was inconsistent between studies, and none of them has been associated both with response, PFS and overall survival (OS). Assessment of circulating VEGF has been reported to be impaired by VEGF bound to bevacizumab and VEGF released from activated platelets in patients with cancer (Niers, Plos one, 2011, 6(5), e19873).

With the use of other antiangiogenic agents such as sunitinib and vandetanib, circulating potential biomarkers of treatment benefit include VEGF-C, soluble VEGFR-3 or change in plasma concentration of VEGF, ICAM-1 and interleukins (Rini et al., J. Clin. Oncol., 2008, 26, 3743-3748; Hanrahan et al., J. Clin. Oncol., 2010, 28, 193-201). However, the magnitude of the association of these biomarkers with PFS and/or response was relatively low, and was not explored for OS.

MMP2 belongs to the matrix metalloproteinase (MMP) family, whose activity has been implicated in proteolysis of extra-cellular matrix, regulation of cell adhesion and migration, processing of growth factors and cytokines, and liberation of angiogenic factors (Roy et al., J. Clin. Oncol., 2009, 27, 5287-5297). MMP, and particularly MMP2 and MMP9, expression in plasma, urine, or tumor tissue has been considered as potential biomarkers which could reflect diagnosis, dissemination and staging, prognosis, and effect of therapy in various cancers. Expression and/or activity of MMP2 and MMP9 in urine, CSF or plasma appear to be correlated to tissue expression in bladder cancer and brain tumors (Papathoma et al., Anticancer research, 2000, 20, 2009-2013; Smith et al., Clin. Cancer Res., 2008, 14, 2378-2386). However, very few studies, restricted to colorectal and prostate cancer, have tested the prognostic or predictive value of MMP2 plasma level. High expression of MMP2 and 9 have been associated to tumor aggressiveness and poor prognosis in various cancers. In high grade glioma, the prognostic value of MMP2 tissue expression is unclear (Jäälinojä, J. Neuro-Oncol, 2000, 46, 81-90; Colin et al., Acta Neuropathol., 2009, 118, 745-754; Brell et al., Brain Tumor Pathol., 2011, 28, 137-144).

In some patients with recurrent high grade glioma (HGG) treatment with bevacizumab has been associated to an initial decrease of urine MMP2 activity, followed by a upregulation at the time of progression in urine (Takano et al., Brain Tumor Pathol., 2010, 27, 89-94) or an overexpression in tumor tissue (de Groot et al., Neuro-Oncol., 2010, 12, 233-242), giving arguments to consider MMP2 as a potential actor of infiltrative escape from bevacizumab. However, baseline MMP2 was not considered is these cases, and it appear that numerous other genes are upregulated after bevacizumab treatment, so that others candidate could be involved in the invasive phenotype associated to tumor progression under bevacizumab (Lucio-Eterovic et al., Clin. Cancer Res., 2009, 15, 4589-4599). With other antiangiogenic agentssuch as VEGFR tyrosine kinase inhibitors like cediranib in GBM, a large panel of plasma biomarkers including MMP2, VEGF, soluble VEGFR-2, placental growth factor (P1GF), SDF1-α, , MMP10 and Ang2 has been evaluated at multiple time point. Various biomarkers including MMP2, VEGF, soluble VEGFR-2 and PlGF present transient variations during treatment that have been related either to progression or survival (Batchelor et al., J. Clin. Oncol., 2010, 28, 2817-2823). For example, cediranib treatment induced a decrease in MMP-2 in plasma, while an early increase in plasma MMP-2 at 8 hours after first administration of cediranib correlated with reduced progression-free survival (PFS) and overall survival (OS). However, none of them, when evaluated at baseline, showed a correlation with PFS or OS.

Therefore, predictive biomarkers that can be used in advance of antiangiogenic therapy to estimate response to therapy and survival of patients are an unmet medical need for patients with cancer.

The inventors have explored the value of potential serological biomarkers to predict response and survival in cancer patients treated with antiangiogenic agents. A set of eleven makers of interest (VEGF, VEGF-R1, fibroblast growth factor (FGF), stromal cell-derived factor 1 (SDF1-α), placental growth factor (PlGF), urokinase plasminogen activator (uPA), plasminogen activator inhibitor-1(PAI1), matrix metalloproteinase 2 (MMP2), matrix metalloproteinase 7 (MMP7), matrix metalloproteinase 9 (MMP9), and adrenomedulline (AM)) were analyzed at baseline and two weeks apart from antiangiogenic therapy initiation in a first cohort of patients treated with bevacizumab based regimen for a recurrent high grade glioma (HGG). Correlations were validated in a separate cohort of patients treated with bevacizumab for a recurrent HGG. Markers analyses were performed in two other cohorts of patients,both treated with cytotoxic agents without bevacizumab, one treated with cytotoxics alone and one treated with cytotoxics and radiotherapy.

Unexpectedly, a high baseline level of MMP2 appears to be predictive of bevacizumab benefit. Among patients with recurrent high grade glioma treated with bevacizumab, but not with cytotoxic agent, higher serum MMP2 level prior to bevacizumab administration was strongly associated with objective response, prolonged tumor control and survival. Therefore, MMP2 appears to be a strong candidate to predict antiangiogenic therapy efficacy in cancer patients.

The present invention relates to the use of matrix metalloproteinase-2 (MMP2) as a predictive biomarker of response to antiangiogenic therapy and survival after antiangiogenic therapy in cancer patients.

The invention relates also to a method for predicting the response to an antiangiogenic treatment and the survival after treatment of a cancer patient, which comprises the step of: measuring the level of MMP2 prior to said antiangiogenic treatment, in a biological sample from said patient, wherein a higher level of MMP2 in said sample, compared to a reference value, is indicative of a response to said antiangiogenic treatment and survival after treatment in said patient.

At the same time, a lower level of MMP2 in said sample, compared to a reference value, is indicative of an absence of response to said antiangiogenic treatment and survival after treatment in said patient.

The method according to the invention is performed on a cancer patient who is to be subjected to an antiangiogenic treatment, to evaluate the efficiency of the antiangiogenic treatment in said patient.

The invention provides for the first time a marker which can predict the efficiency of an antiangiogenic therapy prior to treatment. The MMP2 biomarker of the invention is the only biomarker which allows distinguishing between antiangiogenic treatment responder (high baseline MMP2 level) and non-responder (low baseline MMP2 level) patients and subsequently sorting responder patients, before starting an antiangiogenic therapy. The MMP2 biomarker of the invention has thus the advantage of allowing the selection of the patients in which the antiangiogenic treatment will be efficient.

According to the invention, cancer patients with high MMP2 level prior to antiangiogenic treatment will benefit from the treatment and have a positive treatment outcome. As a consequence, cancer patients with high MMP2 level prior to antiangiogenic treatment will have a prolonged tumor control and survival compared to treated patients having a low level MMP2 level before antiangiogenic treatment and untreated patients.

### Definitions

- Biomarker refers to a distinctive biological or biologically derived indicator of a process, event or condition.
- Predictive biomarker refers to a biomarker that can be used in advance of therapy to estimate response and/or survival of a patient on a specific treatment.
- Predicting a status or event refers to making a finding that has an individual has a significantly enhanced or reduced probability of having a given status or experienced an event.
- Antiangiogenic treatment or antiangiogenic therapy refers to a treatment with an agent, for example a pharmacological agent, which inhibits angiogenesis. Antiangiogenic treatment may be a monotherapy or a combined therapy with one or more anticancer agents such as cytotoxic drugs and cytokines.
- Cancer refers to any malignant solid tumor.
- Cancer patient refers to an individual diagnosed with cancer.
- Response to an antiangiogenic treatment or therapy of a cancer patient refers to a positive medical response to an antiangiogenic treatment characterized by objective parameters or criteria such as objective clinical signs like the reduction of size of the tumor. The objective criteria for determining the response to an antiangiogenic treatment are well-known in the art. The response is generally evaluated according to the revised RECIST 1.1 criteria (Eisenhauer et al., Eur. J. Cancer., 2009, 45, 228-247), although in glioma, response criteria have been recently reassessed as RANO (Wen et al., J. Clin. Oncol., 2010, 28, 1963-1972)..
- Biological sample refers to a biological material likely to contain MMP2. The biological material which may be derived from any biological source is removed from the cancer patient by standard methods which are well-known to a person having ordinary skill in the art.
- Survival, unless otherwise mentioned, refers to the Progression-free survival (PFS) and the overall survival (OS). OS is the length of time that a person lives after being diagnosed with cancer. PFS is the length of time that a person lives free from any significant increase of tumor, after being diagnosed with cancer.
- matrix metalloproteinase-2 or MMP-2, unless otherwise mentioned, refers to the protein or messenger RNA (mRNA) that is encoded by the *MMP2* gene including all allelic variants of said gene. Matrix metalloproteinase-2 (MMP2, MMP 2, MMP-2, MMP-II) is also named as matrix metallopeptidase 2, 72 kDa type IV collagenase, gelatinase A, CLG4A, MONA and TBE-1. The human MMP-2 protein and mRNA sequences correspond respectively to GeneBank Accession Numbers NM_004530 and NP_004521 in the NCBI database. The version of these sequences corresponds preferably to the last version in force on May 23, 2012.
- higher level refers to a significant higher level, i.e., p-value inferior to 0.1.
- reference value refers to a value established by statistical analysis of values obtained from a representative panel of individuals. The panel may for example depend from the nature of the sample, the type of cancer. The reference value can for example be obtained by measuring MMP2 concentrations in a panel of cancer patients non-treated with an antiangiogenic agent (baseline level of MMP2) and determining the median concentration which is used as reference value. When the method according to the invention aims at monitoring a patient, the reference value may be obtained from the patient previously tested.

The method/use of the invention comprises the use of MMP2 alone, in the absence of any other biomarker. According to the invention, the level of a single biomarker, MMP2 alone, prior to angiogenic therapy, is sufficient to predict the efficiency of said therapy and the survival after therapy in cancer patients. The reference value which is used for comparison may be the baseline level of MMP2 obtained by determining the median concentration of MMP2 in a panel of cancer patients not treated with an antiangiogenic agent. The reference value may be obtained from the same type of biological sample and/or from a panel of patients with the same type of cancer, as the tested patient.

In a preferred embodiment of the above identified method/use, said antiangiogenic therapy is with a pharmacological agent which targets the vascular endothelial growth factor (VEGF) pathway.

In a more preferred embodiment, said agent is an anti-VEGF antibody, in particular bevacizumab (Avastin).

In another more preferred embodiment, said agent is a VEGF receptor tyrosine kinase inhibitor (TKI), including a multi (pan)-targeted or a VEGF receptor-targeted TKI. In particular, the VEGF receptor tyrosine kinase inhibitor may be selected from the group consisting of: sunitinib (Sutent), vandetanib (Zactima), pazopanib (Votrient), sorafenib (Nexavar) and cediranib.

In another preferred embodiment of the above identified method/use, said cancer is associated with VEGF overexpression. In a more preferred embodiment, said cancer is selected from the group consisting of: glioblastoma, breast, colon, lung, liver, kidney, pancreas, thyroid and ovarian cancers. In particular, said cancer may be selected from the group consisting of: newly diagnosed and recurrent glioblastoma, metastatic breast cancer, metastatic colorectal cancer, metastatic non-squamous non-small-cell lung cancer (NSCLC), metastatic renal cell carcinoma (RCC), ovarian cancer, advanced pancreatic neuroendocrine cancer, hepatocellular carcinoma, and medullary thyroid cancer.

In another preferred embodiment of the above identified method/use, said patient is a human individual. In particular, said patient is a newly diagnosed individual, not treated with any anticancer drug after cancer diagnosis.

In another preferred embodiment of the above identified method, said biological sample is a body fluid or biopsied tumor cells or tissue. The body fluid may be serum, plasma, blood, lymph, synovial, pleural, peritoneal, or cerebrospinal fluid, mucus, bile, urine saliva, tears and sweat. Preferably, the biological sample is a body fluid, in particular plasma, serum or urine.

MMP2 level may be assayed directly on the biological sample or following a standard pretreatment, according to pretreatment methods which are well-known to a person having ordinary skill in the art in the art. Pretreatment may include for example preparing plasma from blood, diluting viscous fluids, lysing cells, extracting and precipitating RNA, and embedding biopsied tissue in plastic or paraffin.

MMP2 level can be measured using a variety of techniques for detecting and quantifying the expression of a gene or the activity of a gene product, that are well-known to a person having ordinary skill in the art. Such techniques typically include methods based on the determination of the level of transcription (i.e., the amount of mRNA produced), methods based on the quantification of the protein encoded by the *MMP2* gene, and methods based on the quantification of the enzymatic activity of the MMP2 protein.

In another preferred embodiment of the above identified method, it comprises measuring MMP2 messenger RNA (mRNA) level in said biological sample, preferably biopsied tumor cells or tissue.

MMP2 mRNA level may be measured, either by hybridization to a specific probe, eventually labeled with a detectable label and/or immobilized on the surface of a solid support (plate, slide, strip, wells, microparticles, fiber, gel), or by amplification using specific primers, eventually labeled with a detectable label. Preferably, the MMP2 mRNA level is measured using an assay selected from the group consisting of: nucleic acid array- or tissue microarray-based assay, and quantitative reverse transcription polymerase chain reaction (qRT-PCR) assay. One skilled in the art will know which parameters may need to be manipulated to optimize detection and/or quantification of the MMP2 mRNA using these techniques.

In another preferred embodiment of the above identified method, it comprises measuring MMP2 protein level in said biological sample, preferably a body fluid, more preferably plasma, serum or urine.

Measurement of MMP2 protein level may be achieved using several different techniques, many of which are antibody-based. Example of such techniques include with no limitations immunoassays (Enzyme-linked immunoassay (ELISA), radioimmunoassay, chemiluminescence- and fluorescence-immunoassay), immunohistochemistry assays and antibody microarray-based assays. Preferably, MMP2 protein level is measured using an immunoassay such as ELISA. MMP2 antibodies are well-known in the art and various monoclonal and polyclonal antibodies are available, including mouse, rabbit and sheep polyclonal antibodies and various mouse monoclonal antibodies (clone 4D3, 2C1, 8B4, 42-5D11). One skilled in the art will know which parameters may need to be manipulated to optimize detection and/or quantification of the MMP2 protein with MMP2 antibodies, using these techniques.

In yet another preferred embodiment of the above identified method, it comprises measuring MMP2 enzymatic activity level in said biological sample, preferably a body fluid, more preferably, plasma, serum or urine.

MMP2 enzymatic activity (gelatinolytic activity) may be measured by gelatin zymography, according to protocols which are well-known in the art (Yamamoto et al., Cancer Res., 1996, 56, 384-392; Uemura et al., Circ.; Res., 2001, 88, 1291-1298).

The method according to the present invention may be performed simultaneously or subsequently on biological samples from different patients.

The above mentioned method may further comprise, after the measuring step, a further step of sorting the cancer patient(s) into responder or non-responder based on MMP2 level(s) in said biological sample(s).

A particularly advantageous embodiment of the present invention is the use of MMP2 protein as a predictive biomarker of response to an anti-VEGF antibody therapy, in particular bevacizumab (Avastin) therapy, and survival after therapy in patients with glioblastoma, in particular recurrent glioblastoma. The embodiment comprises preferably the use of MMP2 protein level in a body fluid, more preferably, plasma, serum or urine, as a predictive biomarker of said response/survival.

Another particularly advantageous embodiment of the present invention is a method for predicting the response to an anti-VEGF antibody treatment, in particular bevacizumab (Avastin) treatment, and the survival after treatment of a patient with glioblastoma, in particular recurrent glioblastoma, which comprises the step of: measuring the level of MMP2 protein prior to said anti-VEGF antibody treatment, in a serum or plasma sample from said patient, wherein a higher level of MMP2 in said sample, compared to the median serum MMP2 concentration at baseline in a panel of cancer patients non-treated with an antiangiogenic agent, is indicative of a response to said anti-VEGF antibody treatment and survival after treatment in said patient.

The invention relates also to a method for monitoring the response to an antiangiogenic treatment of a patient suffering from cancer, comprising: measuring the level of MMP2 in a biological sample from the patient, at one or more time points during said angiogenic treatment, wherein an equal or higher level of MMP2 in said sample, compared to a reference value, is indicative of a prolonged response to said antiangiogenic treatment, whereas a lower level of MMP2 is indicative of a resistance to said angiogenic therapy.

The reference value may be the same as for the above mentioned method for predicting the response to an antiangiogenic treatment. Alternatively, the reference value may be the baseline level of MMP2, in the same type of biological sample from the same patient, prior to said angiogenic treatment.

The method(s)/use according to the invention are not carried out *in vivo,* but *in vitro* and/or *ex vivo.*

The practice of the present invention will employ, unless otherwise indicated, conventional techniques which are within the skill of the art. Such techniques are explained fully in the literature. See, for example, Current Protocols in Molecular Biology (Frederick M. AUSUBEL, 2003, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); The immunoassay Handbook (D. Wild, ed., , Elsevier LTD, Oxford, 3rd ed. May 2005).

In addition to the preceding features, the invention further comprises other features which will emerge from the description which follows, which refers to examples illustrating the methods and uses according to the present invention, as well as to the appended drawings in which:

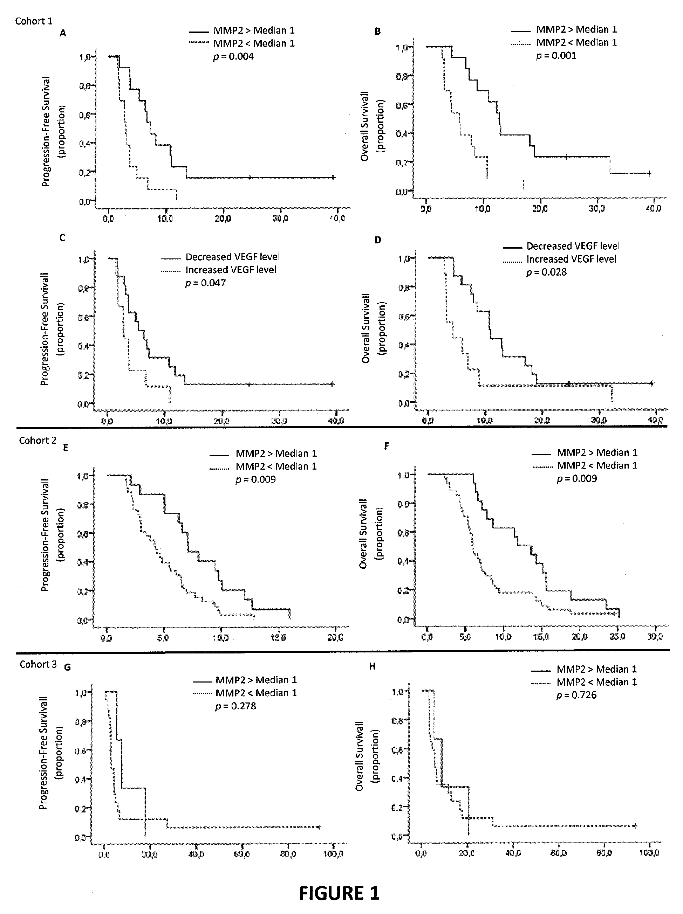

- figure 1 presents survival analyses. A and B: Progression-Free Survival (PFS) and Overall Survival (OS) of serie 1 patients according to plasmatic MMP2 level. C and D: PFS and OS of serie 1 patients according to evolution of plasmatic VEGF level. E and F: PFS and OS of serie 2 patients according to plasmatic MMP2 level, dichotomized by serie 1 MMP2 median. G and H: PFS and OS of serie 3 patients according to plasmatic MMP2 level, dichotomized by serie 1 MMP2 median (Median 1). A, B, E, F, G, H: —: MMP2 > Median 1; ...... : MMP2 <Median 1. C, D: —:Decreased VEGF level; ...... : Increased VEGF level.

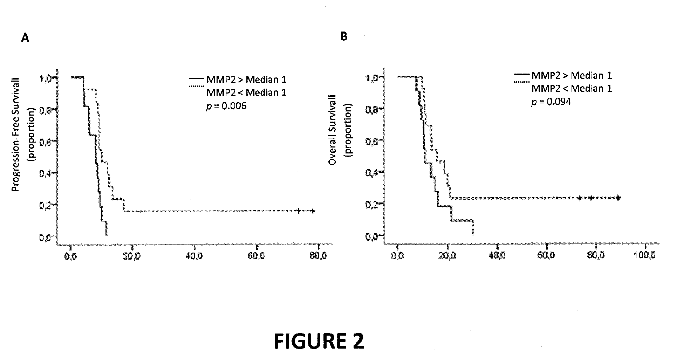

- figure 2 presents survival analyses: Progression-Free Survival (A) and Overall Survival (B) of serie 4 patients according to plasmatic MMP2 level, dichotomized by serie 1 MMP2 median. A, B: —: MMP2 > Median 1; ...... : MMP2 <Median 1.

### Example 1 : Material and methods

### Patients

All patients were recruited from the University Timone Hospital in Marseilles from to July 2007 to March 2010 (cohort 1 and 2); from June 2003 to February 2007 (cohort 3), and from September 2004 to May 2007 (cohort 4). Eligible patients included those patients aged 18 years or older with recurrent high grade glioma treated with bevacizumab at least 3 months apart from the end of radiotherapy. Treatment regimens, evaluation and follow-up were similar for cohort 1 and 2. At the time of bevacizumab initiation administrated for recurrence or progression of their disease, patients were proposed to participate to that study. Plasma was collected before bevacizumab dose administration. All patients were informed of the investigational nature of the study and provided written informed consent in accordance with institutional and national guidelines. This protocol was approved by institutional review board.

### Initial cohort (cohort 1)

Cohort 1 included 26 patients with recurrent HGG for which, at least, 2 dosages were available: a baseline time point collected before first dose administration and another point at day 15, just before the second dosing of bevacizumab. All patients were treated with the combination of bevacizumab 10mg/kg and irinotecan 340 mg/m2 (if taking enzyme-inducing antiepileptic drugs) or 125mg/m2 (if no taking enzyme-inducing antiepileptic drugs) every 2 weeks. The characteristics of the 26 patients included are described in Table I. Diagnosis of progression that motivates bevacizumab treatment was based on clinical and magnetic resonance imaging (MRI) data, completed by fluorodeoxyglucose positron emission tomography (FDG-PET) imaging if needed by the referring physician. An interval of 3 months after radiotherapy was required before bevacizumab initiation to avoid pseudo-progression. None of the patients had histologic confirmation of recurrence, so that histology reported is the first documented histology for each patient. First documented histology was glioblastoma for 20 patients (76.9%); all were treated upfront with radiotherapy and temozolomide. Bevacizumab and irinotecan was applied in second, third, and fourth line for 13, 6, and one patients, after gliadel or BCNU (carmustine). Six patients presented an initial histology of a mixed oligoastrocytoma grade III. Up front treatment were procarbazine, lomustine (CCNU) and vincristine (PCV) and radiotherapy (n=4) or BCNU, temozolomide and radiotherapy (n=2). Bevacizumab and irinotecan was applied in third or fourth line after temozolomide, gliadel or carboplatine etoposide. Duration of follow-up was 24.6 and 39. At the time of last follow-up, April 15, 2012, 24 patients died of disease.

### Cohort 2

Patients for whom only baseline time point was available constitute the second cohort. 50 patients were included with similar characteristics although 40% exhibit a poor performance score (Karnovsky performance score (KPS) ≤ 60) at the time of bevacizumab initiation, versus 15.4% in the first cohort. Thirty one patients (62%) had a documented initial histology of GBM and were also treated with radiotherapy and temozolomide upfront. Bevacizumab and irinotecan was administrated as second line treatment in 19, third line treatment in 11 and fourth line treatment in one patient. Among patients with initial grade II or III tumor, 19 received bevacizumab based regimen as the second line in 11 % (n=2), third line in 68% (n=13) and the fourth line in 21 %(n=4) . At the time of last follow-up, April 15, 2012, all patients died of disease.

### Cohort 3

In view of the results observed, a third cohort of patients with GBM treated with alkylating based chemotherapy regimen without any further administration of bevacizumab in previous or subsequent lines, was retrospectively identified from a plasma collection. Since serum was rarely available at the time of recurrence, patients treated with upfront or exclusive chemotherapy were selected, i.e. patients with bulky disease treated with the combination of BCNU and temozolomide (n=18) or elderly patients inoperable tumor and poor KPS treated with temozolomide as exclusive treatment (n=2). In all cases, plasma was collected prior to the first administration of chemotherapy. All patients had measurable disease at the time of treatment initiation. Duration of follow-up was 93.4 months. At the time of last follow-up, April 15, 2012, 19 patients died of disease.

### Cohort 4

In view of the results observed, a fourth cohort of patients with GBM, treated with the standard regimen defined in the first line setting that combine chemotherapy (temozolomide) and radiotherapy without any further administration of bevacizumab in previous or subsequent lines, was retrospectively identified from a plasma collection. In all cases, plasma was collected prior to the first administration of chemotherapy. All patients are evaluable for PFS and OS.

### Clinical follow-up

Clinical follow-up of patients was performed every 4 weeks. MRI was performed every 8 weeks, according to our local guidelines. Responses were assessed according to RANO criterias, that incorporate FLAIR sequence as part of the evaluation to take into account infiltrative progression as observed with antiangiogenic agents. All responses were confirmed on subsequent MRI, two months apart. All responses observed in cohort 1 and 2 were reviewed.

### Plasma markers assay

Plasma samples were collected from patients before cycle 1, after completion of cycle 1 and when available, at the time of progression. Peripheral blood was drawn into a citrated Vacutainer® tube, mixed immediately and centrifuged within 30 minutes of collection. Plasma was removed and was transferred to cryogenic storage tubes. Samples were stored immediately at -80°C.

Samples were analyzed for levels of vascular endothelial growth factor (VEGF), vascular endothelial growth factor receptor 1 (VEGF R1), Placenta growth factor (PlGF), Fibroblast growth factor (FGF), stromal cell-derived factor 1 (SDF 1), urokinase plasminogen activator (u-PA), plasminogen activator inhibitor-1 (PAI-1), matrix metalloproteinase 2 (MMP2), matrix metalloproteinase 7 (MMP7), matrix metalloproteinase 9 (MMP9) and adrenomedulline (AM), using commercially available enzyme-linked immunosorbent assay (ELISA) kits (R&D Systems). Samples were run in duplicate, and the average was recorded.

### Statistical analysis

Categorical variables were summarized as frequencies and corresponding percentages and continuous variables as median and range. Overall survival (OS) was defined to be time from randomization to death from any cause, censored at the date of last contact. Progression Free Survival (PFS) was time from randomization to documented progression per RANO criteria or death, censored at the date of the last documented disease evaluation for patients without a PFS event reported. The Kaplan-Meier method was used to estimate survival and PFS distributions. Log-rank tests were used for univariate comparisons of OS and PFS end points. Cox proportional hazards models were used for multivariate analyses and to estimate hazard ratios in regression models. The reported *p*-values are two-sided, and *p*<0.05 was considered statistically significant. Responses were dichotomized into responders (best response of partial or complete response per RANOcriteria) and nonresponders (stable disease or progresion). Subjects were divided into two groups based on their baseline biomarkers levels using the median value as the cutoff. Within each of the biomarkers groupings, a Fisher exact test with a two-sided 5% type I error rate was used to detect an association between response and treatment. Mann-Whitney U-test was used to detect an association between response and continuous value of biomarkers. Calculation sensitivity and specificity of MMP2 cuttoff in the determination of response was performed using receiver operating characteristic (ROC) curve analysis. Survival status was updated in April of 2012.

### Example 2 : High serum MMP2 baseline level correlates with response and survival in patients treated with bevacizumab for recurrent high grade glioma.

Given remarquable but heterogeneous activity of bevacizumab, particularly in glioblastoma, the inventors have explored the value of potential serological biomarkers to predict response and survival in patients treated with bevacizum for a recurrent high grade glioma (HGG).

A set of eleven makers of interest (VEGF, VEGF-R1, FGF, SDF1-α, PlGF, uPA, PAIl, MMP2, MMP7, MMP9, and adrenomedulline (AM)) were analyzed, using ELISA, at baseline and two weeks apart from bevacizumab initiation in a first cohort of 26 patients treated with bevacizumab based regimen in University Timone Hospital (Marseille, France), for a recurrent HGG between July 2007 and March 2010 (cohort 1); date of last follow-up was April 2012. Correlations were validated in a separate cohort of 50 patients from the same institution treated with bevacizumab for a recurrent HGG (Cohort 2) and then tested in two other cohorts of patients, a third cohort of 20 patients treated with cytotoxic agents (Cohort 3) and a fourth cohort of 24 patients treated with cytotoxic agents and radiotherapy (Cohort 4), both without bevacizumab. The characteristics of the patients included in the study are described in Table I.

**Table I : Characteristics of patient populations**

| | **Serie 1 (n=26)** | | **Serie 2 (n=50)** | | **Serie 3 (n=20)** | | **Serie 4 (n = 24)** | |
|---|---|---|---|---|---|---|---|---|
| | **No of patients %** | | **No of patients %** | | **No of patients %** | | **No of patients %** | |
| **Age** | 56.1 (22.3-73.2) 16 men / 10 women | | 59.7 (18.3-76.7) 34 men / 16 women | | 56.1 (44.4-76.8) 14 men / 6 women | | 62.2 (37.7-72.6) 12 men / 12 women | |
| **Gender** | | | | | | | | |
| **Histology** | | | | | | | | |
| **Grade II** | 0 | 3.8 | 5 | 10 | 0 | 0 | 0 | |
| **Anaplastic** | 6 | 19.2 | 14 | 28 | 0 | 0 | 0 | |
| **Glioblastoma** | 20 | 76.9 | 31 | 62 | 20 | 100 | 24 | 100 |
| **Response** | 12 | 48 | 18 | 36.7 | 5 | 25 | | |
| **Complete response** | 3 | 12 | 1 | 2 | 1 | 5 | | |
| **Partial response** | 9 | 36 | 17 | 34.3 | 4 | 20 | | |
| **Absence of response** | 13 | 52 | 31 | 63.3 | 15 | 75 | | |
| **Stable disease** | 2 | 8 | 15 | 30.6 | 5 | 25 | | |
| **Progression** | 11 | 44 | 16 | 32.7 | 10 | 50 | | |
| **Non evaluable** | 1 | | 1 | | 0 | | | |
| **Treatment line** | | | | | | | | |
| **1** | 0 | 0 | 0 | 0 | 20 | 100 | 24 | 100 |
| **2** | 15 | 57.5 | 21 | 46 | | | | |
| **3** | 7 | 26.9 | 24 | 48 | | | | |
| **4** | 3 | 11.5 | 4 | 4 | | | | |
| **5** | 0 | | | 1 2 | | | | |
| **6** | 1 | 3.8 | | | | | | |
| **KPS** | | | | | | | | |
| **50** | 0 | | | | 2 4 1 | 5 | 0 | |
| **60** | 4 | 15.4 | 18 | 36 | 7 | 35 | 2 | |
| **70** | 15 | 57.7 | 18 | 26 | 7 | 35 | 6 | |
| **80** | 7 | 26.9 | 12 | 24 | 5 | 25 | 14 | |
| **90** | | | | | | | 2 | |
| **OS (months)** | 8.7 | | 7.1 | | 6.2 | | 13.5 (8.7-17.9) | |
| **Responders** | 13 | | 14.6 | | 20.6 | | | |
| **Non responders** | 4.5 | | 5.8 | | 5.4 | | | |
| **PFS (months)** | 4.4 | | 5.3 | | 4.2 | | 9.1 (8.7-9.5) | |
| Responders | 8.2 | | 8 | | 17.9 | | | |
| Non responders | 2.8 | | 3 | | 2.7 | | | |

Plasma marker dosages were correlated to objective response as analyzed by RANO criteria's, Progression-free survival (PFS), and overall survival (OS).

### 1) Results

### Initial cohort (cohort 1)

In the original patient data set (n=26 patients), response could be evaluate for 25 patients (Table I). Twelve patients (48%) exhibit an objective response either complete (n=3) either partial (n=9), while 13 patients (52%) were either stable for at least 2 months (n=2) or progressive (n=11). Responses were durable, since median PFS was of 8.2 months in responding patients (IC95: 2.3-14.0) versus 2.8 months for non responders (IC95:1.6-4.1) (p<0.001); responses were correlated to survival with a median overall survival of 13 months in responders (IC95: 5.8-20.1) versus 4.5 months for non responders (IC95: 2.7-6.2) (p < 0.001). Median PFS of that population was of 4.4 months (IC95: 2.1-5.5) with a median overall survival of 8.7 months (IC95: 5.3-11.7).

Biomarkers kinetics after the first bevacizumab administration was characterized by a decrease of PlGF levels in all patients tested while other markers exhibit an heterogeneous variation at day 15. VEGF decreased in 16 out of 25 patients while VEGFR increased in 18 out of 26 patients. MMP2 and MMP9 increase respectively in 10 and 6 out of 25 patients.

Association of biomarkers and bevacizumab treatment outcome was first analyzed for baseline levels. In univariate analysis, a strong correlation was observed for MMP2 and MMP9 levels with objective response, progression free survival and overall survival. Among the 12 patients with high MMP2 level, 10 (83.3%) responses were observed, while in the 13 patients with low MMP2 level, only 2 (15.4%) patients experienced an objective response (p=0.001; Table II).

**Table II : Response rates according to patients cohorts and plasmatic MMP2 level**

| | Serie 1 | | | Serie 2 | | | Serie 3 | | |
|---|---|---|---|---|---|---|---|---|---|
| | Responders (R) | Non Responders (NR) | Response-Rate (RR) | R | NR | RR | R | N R | RR |
| MMP2 < | | | | | | | | 1 | |
| Median serie 1 | 2 | 11 | 15.4% | 6 | 28 | 17.6% | 4 | 3 | 24% |
| MMP2 > | | | | 1 | | | | | |
| Median serie 1 | 10 | 2 | 83.3% | 2 | 3 | 80% | 1 | 2 | 33.3% |
| *P* value | | | **0.001** | | | **< 0.0001** | | | 0.601 |

This correlation between MMP2 and response remains significant if the value of MMP2 level was considered as a continuous variable (p=0.005). Inversely, a low MMP9 level was associated with higher probability of response, with 8 OR observed out of 11 patients (72.7 %), versus 4 OR out of 13 patients (30.8%) with a high MMP9 level (p = 0.041). However, considering MMP9 as a continuous variable, correlation between MMP9 and response is not confirmed (p = 0.094). ROC curve analysis was performed in order to evaluate the performance of plasma MMP2 levels in discriminating between responders and non responders. Plasmatic MMP2 level had a high discrimination value with an area under curve of 0.827 (IC95%: 0.624-0.947; p = 0.0017). With a cutoff value of 227.5 ng/ml, the sensitivity was 83.3 % (IC95% 50.9-97.1) and the specificity was 84.6 % (IC95% 53.7-97.3).

In univariate analysis, MMP2 significantly impact both PFS (p=0.004) and OS (p=0.001) (Table III and figure 1), as did MMP9 (p=0.007 for PFS; p=0.015 for OS; Table III).

**Table III: Univariate and multivariate analyses of progression free survival (PFS) and overall survival (OS) according to median plasmatic biomarkers**

| | | **PFS** | | | **OS** | |
|---|---|---|---|---|---|---|
| **Plasmatic** | **univariate** | **multivariate** | | **univariate** | **multivariate** | |
| **biomarkers** | *p* value | *p* value | hazard ratio | *p* value | *p* value | hazard ratio |
| VEGF | 0.255 | | | 0.263 | | |
| VEGF evolution | **0.047** | **0.033*** | **2.822** (1.088-7.321) | **0.028** | **0.021*** | **3.170** (1.193-8.422) |
| VEGF R1 | 0.789 | | | 0.6 | | |
| VEGF R1 evolution | 0.191 | | | 0.447 | | |
| PlGF | 0.195 | | | 0.475 | | |
| FG F | 0.841 | | | 0.904 | | |
| FGF evolution | 0.692 | | | 0.543 | | |
| SDF1 | **0.046** | **0.068*** | **2.267** (0.942-5.456) | **0.101** | | |
| SDF1 evolution | 0.951 | | | 0.966 | | |
| uPA | 0.063 | | | **0.016** | **0.004*** | **4.289** (1.598-11.516) |
| uPA evolution | 0.612 | | | 0.982 | | |
| PAI1 | 0.408 | | | 0.389 | | |
| PAI1 evolution | 0.627 | | | 0.565 | | |
| MMP2 | **0.004** | **0.007*** | **3.925** (1.465-10.517) | **0.001** | **0.005*** | **4.618** (1.577-13.527) |
| MMP2 evolution | 0.672 | | | 0.621 | | |
| MMP7 | 0.121 | | | 0.259 | | |
| MMP7 evolution | 0.754 | | | 0.493 | | |
| MMP9 | **0.007** | **0.016*** | **4.290** (1.306-14.084) | **0.015** | **0.025*** | **3.487** (1.170-10.390) |
| MMP9 evolution | 0.303 | | | 0.42 | | |
| AM | 0.429 | | | 0.401 | | |
| AM evolution | 0.748 | | | 0.763 | | |

| | | | | | | |
|---|---|---|---|---|---|---|
| * adjusted by age and kamofsky performans status | | | | | | |

Patients with initial high level of MMP 2 presented a median PFS of 7.3 months (IC95: 5.2-9.4) and a median OS of 12.8 months (IC95: 10.4-15.2) as compared to a median PFS of 3.0 months (IC95: 2.5-3.5) and a median OS of 5.9 months (IC95: 4.0-7.8) in case of initial low MMP2 level. Patients with initial low level of MMP 9 presented a median PFS of 8.2 months (IC95: 1.4-15.0) and a median OS of 12.3 months (IC95: 0 - 26.1) as compared to a median PFS of 3.7 (IC95: 2.9-4.6) and a median OS of 6.9 (IC95: 4.6-9.3) in case of initial high MMP9 level. uPA and SDF1 were only correlated either to overall survival (p=0.016) or PFS (p=0.046) respectively. No other markers baseline levels had a statistically significant relation with response, PFS and OS (Table III). Other factors, including age, KPS, histology, and number of previous lines, had also no significant impact on outcome in cohort 1. In a multivariate Cox regression model that included baseline biomarkers levels, and potential prognostic factors (age and KPS), baseline level of MMP2 and MMP9 remained significant for PFS (hazard ratio, 3.925; 95% CI 1.465-10.517; p=0.007 for MMP2 and hazard ratio, 4.290; 95% CI 1.306-14.084; p=0.016 for MMP9) and for OS (hazard ratio 4.618; 95% CI 1.577-13.527; p=0.005 for MMP2 and hazard ratio, 3.487; 95% CI 1.170-10.390; p=0.025 for MMP9).

Association of biomarkers kinetics in the first month and bevacizumab treatment outcome was then analyzed. In univariate analysis, only initial kinetics of VEGF was significantly correlated with outcome, both for PFS (p=0.047) and OS (p=0.021). Patients with an initial decrease of VEGF presented a median PFS of 5.4 months (IC95: 2.5-8.2), as compared to a median PFS of 2.8 months (IC95: 2.7-3.0), in case of initial VEGF decrease. Median OS for initial decreased and increased VEGF level patients were 10.7 (IC95: 7.5-13.9) and 4.4 months (IC95: 1.0-7.7) respectively. No other biomarker showed correlation between early change and outcome. In the multivariate Cox regression model that included initial kinetics biomarkers levels, age and KPS, VEGF initial change remains significant for PFS (hazard ratio 2.822; 95% IC95: 1.088-7.321; p=0.033) and OS (hazard ratio 3.170; 95% CI 1.193-8.422; p=0.021; Table III and figure 1).

### Cohort 2

In view of the results in our initial cohort, a second cohort of 50 patients treated with bevacizumab and irinotecan for a recurrent HGG for whom plasma was available at baseline only, before bevacizumab administration, was identified. Objective response rate in this less selected population was of 36.7% including complete response in 2% and partial response in 34.3%. PFS and OS observed in responding patients were similar to the ones observed in cohort 1 (8 months and 14.6 months respectively). Median PFS of cohort 2 was of 5.3 months (IC95: 3.4-6.7), and median OS was of 7.1 months (IC95: 5.9-8.2) (Table I).

Baseline MMP2 and MMP9 were the only biomarkers assessed in that cohort. The cut-off for MMP2 and MMP9 defined in cohort 1 were applied for subsequent analysis. In that cohort, 16 (32 %) patients exhibit a high MMP2 level and 27 (54 %) patients a low MMP9 level. Response could be evaluated for 49 patients. MMP2 similarly impacted response rate in that population, with 12 objective responses (RR: 80%) observed in the 15 patients with high MMP2 level and 6 objective responses (RR: 17.6%) in patients with low MMP2 level (p < 0.0001; Table II). High plasmatic MMP2 level was associated to a median PFS and OS of 7.1 (IC95%: 5.3-8.9) and 11.8 (IC95%: 7.6-16.1) versus 4.2 (IC95%: 2.9-5.5) and 5.9 months (IC95%: 5.4- 6.4), in cases with low MMP2 level (p=0.009 for PFS and p=0.009 for OS; figure 1). No correlation was observed between MMP9 level and PFS or OS in cohort 2.

### Cohort 3

Objective response rate in this population was of 25% including complete response in 5% and partial response in 20%. Median PFS of cohort 3 was of 4.2 months (IC95: 1.7-6.6), and median OS was of 6.2 months (IC95: 3.7-7.9) (Table I). PFS and OS observed in responding patients were significantly higher than in non responding patients (p = 0.002 for OS and p = 0.005 for PFS). Baseline MMP2 and MMP9 were the only biomarkers assessed in that cohort. The cut-off for MMP2 and MMP9 defined in cohort 1 were again applied for subsequent analysis. In that cohort, 3 (15 %) patients exhibit a high MMP2 level and 12 (60 %) patients a low MMP9 level. MMP2 did not impact response rate in that population, with 1 and 4 objective responses in case of high and low MMP2 level respectively (p=0.601). There was no association between MMP 2 baseline level and PFS (p=0.278) or OS (p = 0.726; figure 1). No correlation was observed between MMP9 level and PFS (p = 0.335), OS (p = 0.490) or responses (p = 0.601) in cohort 3.

### Cohort 4

Response could not be evaluated in that cohort of patients that underwent surgical removal of the tumor. Median PFS and OS were of 9.1 (95%CI: 8.7-9.5) and 13.5 (95%CI: 8.7-17.9) respectively.

Baseline MMP2 and MMP9 were the only biomarkers assessed in that cohort. The cut-off for MMP2 and MMP9 defined in cohort 1 were again applied for subsequent analysis. In that cohort (n=24), 11 patients (46%) presented a high MMP2 level. Opposite to our observation in cohort 1 and 2, a high level of MMP2 was correlated to a shorter PFS (p=0.008) and OS (p=0.047) in multivariate analysis.

**Table IV: Univariate and multivariate analyses of progression free survival (PFS) and overall survival (OS) according to median plasmatic biomarkers**

| | PFS | Univariate Multivariate | | HR | OS | Univariate Multivariate | | HR |
|---|---|---|---|---|---|---|---|---|
| **MMP2** | | 0.006 | 0.008 | 3.821 (1.417-10.305) | | 0.094 | 0.047 | 2.479 (1.014-6.062) |
| Low | 10.0 (6.7-13.2) | | | | 15.7 (9.4-22.1) | | | |
| High | 8.2 (5.3-11.1) | | | | 10.9 (7.7-14.2) | | | |
| MMP9 | | 0.214 | | | | 0.251 | | |
| Low | 8.8 (7.7-9.9) | | | | 11.2 (8.2-14.1) | | | |
| High | 9.1 (8.5-9.7) | | | | | | | |

There was no association between MMP 2 baseline level and PFS (p=0.006) or OS (p = 0.094) in cohort 4 (Table IV and figure 2). No correlation was observed between MMP9 level and PFS (p = 0.214) or OS (p = 0.251) in cohort 4 (Table IV).

### 2) Conclusions

In 2 cohorts of patients with recurrent high grade glioma, treated with bevacizumab based regimen and evaluated in the same institution, higher MMP2 baseline plasma levels were associated with increased response rate, progression free survival and overall survival, after adjustment with age and KPS score. Despite the small number of patients assessed in these 2 cohorts, the magnitude of this effect was highly significant and, both for RR, PFS and OS, similar in each of the cohorts tested, and between the two cohorts.

A higher proportion of objective response (48%) were observed in cohort 1, as compared to cohort 2 (36.7%), which included less selected patients, particularly in regard to KPS (84.6% and 50% of patients in cohort 1 and 2 respectively had a KPS ≥70). However, in these two cohorts of patients treated with bevacizumab, survival associated to objective response was similar between the two cohorts (13 and 14.6 months), and clearly superior to the survival observed in non-responder patients (4.5 and 5.8 months). The strength of the response evaluation may have reinforced the strong correlation which was observed between MMP2 levels and response to bevacizumab.

Taken together, and considering the similar magnitude of association of MMP2 with response, progression-free survival, and survival, these results support the fact that MMP2 plasma level appear to be a robust candidate to predict outcome of patients treated with bevacizumab for an high grade glioma.

This association was not observed in two cohorts of patients treated with cytotoxic agents, with or without radiotherapy and excluding bevacizumab, demonstrating the specificity of MMP2 as a predictive biomarker of antiangiogenic therapy efficiency.

For other potential biomarkers that were tested, MMP9 baseline level exhibited inconsistent results between the 2 cohorts while VEGF kinetic, which impacts PFS and OS in the cohort 1, could not be assessed in the cohort 2. Consistent with previous studies, an increase of PlGF was observed in all patients analyzed, while change of others potential biomarkers tested was heterogeneous. However none of these change appear to influence outcome as observed in most studies with bevacizumab.

This study, the first to demonstrate that high MMP2 plasma level is a predictive biomarker of antiangiogenic therapy benefit, may allow the selection of patients most likely to benefit from bevacizumab treatment, since patients with low MMP2 plasma level appear to experience little benefit if any. Patients with low MMP2 plasma level have a 15% probability of objective response to bevacizumab with an expected median PFS and OS of 3.0 months and 5.9 months. These patients could be offer new investigational agents early in the course of their disease. Since bevacizumab is currently under investigation in the upfront setting of patients with newly diagnosed GBM in large placebo control phases III trial, assessment of MMP2, MMP9, and VEGF initial kinetics is required to reinforce this finding.

## Claims

1. Use of matrix metalloproteinase-2 (MMP2) as a predictive biomarker of response to antiangiogenic therapy and survival after antiangiogenic therapy in cancer patients.

2. The use according to claim 1, wherein said antiangiogenic therapy is with a pharmacological agent which targets the vascular endothelial growth factor (VEGF) pathway.

3. The use according to claim 2, wherein said agent is an anti-VEGF antibody.

4. The use according to claim 2, wherein said agent is a VEGF receptor tyrosine kinase inhibitor.

5. The use according to any one of claims 1 to 4, wherein said cancer is associated with VEGF overexpression.

6. The use of claim 5, wherein said cancer is selected from the group consisting of: glioblastoma, breast, colon, lung, liver, kidney, pancreas, thyroid and ovarian cancers.

7. A method for predicting the response to an antiangiogenic treatment and the survival after treatment of a cancer patient, which comprises the step of: measuring the level of MMP2 prior to said antiangiogenic treatment, in a biological sample from said patient, wherein a higher level of MMP2 in said sample, compared to a reference value, is indicative of a response to said antiangiogenic treatment and survival after treatment of said patient.

8. The method according to claim 7, wherein said biological sample is a biological fluid or biopsied tumor cells or tissue.

9. The method according to claim 8, wherein said biological fluid is serum, plasma or urine.

10. The method according to any one of claims 7 to 9, wherein said MMP2 is MMP2 protein.

11. The method according to claim 7 or 8, wherein said MMP2 is MMP2 mRNA.

12. The method according to any one of claims 7 to 10, which comprises measuring the level of MMP2 protein using an immunoassay.

13. The method according to any one of claims 7, 8 and 11, which comprises measuring the level of MMP2 mRNA using a nucleic acid array-based, a tissue microarray-based or a quantitative reverse transcription polymerase chain reaction assay.

14. The method according to any one of claims 1 to 13, which comprises, after the measuring step, a further step of sorting the cancer patient into responder or non-responder based on MMP2 level in said biological sample.

15. A method for monitoring the response to an antiangiogenic treatment of a patient suffering from cancer, comprising: measuring the level of MMP2 in a biological sample from the patient, at one or more time points during said angiogenic treatment, wherein an equal or higher level of MMP2 in said sample, compared to a reference value, is indicative of a prolonged response to said antiangiogenic treatment, whereas a lower level of MMP2 is indicative of a resistance to said angiogenic therapy.
